# EUROPÄISCHE PATENTSCHRIFT

(11) **EP 1 395 296 B1**
(45) Veröffentlichungstag und Bekanntmachung des Hinweises auf die Patenterteilung: **16.03.2005**
(21) Anmeldenummer: 02748596.0
(22) Anmeldetag: 14.06.2002
(51) Int. Cl.: A61L 24/00, A61L 27/46, A61L 27/44, A61K 6/033, A61K 6/083

(54) **VERFAHREN ZUR HERSTELLUNG EINES BIOAKTIVEN KNOCHENZEMENTS UND KNOCHENZEMENT-KIT**
METHOD FOR PRODUCING A BIOACTIVE BONE CEMENT AND BONE CEMENT KIT
PROCEDE DE PRODUCTION D'UN CIMENT A OS BIOACTIF ET ENSEMBLE CIMENT A OS

(30) Priorität: 15.06.2001 DE 10129842
(43) Veröffentlichungstag der Anmeldung: 10.03.2004
(73) Patentinhaber: Humboldt Universität Berlin Charite Universitätsklinikum (CCM), 10117 Berlin (DE); BAM Bundesanstalt für Materialforschung und -prüfung, 12205 Berlin (DE); Vita Zahnfabrik H. Rauter GmbH & Co. KG, 79704 Bad Säckingen (DE)
(72) Erfinder: MÜLLER, Wolf-Dieter, 12524 Berlin (DE); NAGEL, Emil, 79713 Bad Säckingen (DE); BERGER, Georg, 16341 Zepernick (DE)
(74) Vertreter: Walter, Wolf-Jürgen
(86) Internationale Anmeldenummer: PCT/DE2002/002228
(87) Internationale Veröffentlichungsnummer: WO 2002/102427

(56) Entgegenhaltungen:
- EP-A- 0 383 595
- EP-A- 0 434 010
- WO-A-01/41713
- DE-A- 2 906 413
- DE-A- 19 641 775
- DE-A- 19 963 251
- DE-C- 19 744 809
- GB-A- 1 091 476

## Beschreibung

Die Erfindung betrifft ein Verfahren zur Herstellung eines bioaktiven Knochenzements und einen Knochenzement-Kit zur Verankerung künstlicher Gelenke sowie zum Ausfüllen von Knochendefekten.

Knochenzemente für die Gelenkverankerung und für sonstige Knochendefekte bestehen aus einem Kunststoff, in der Regel basierend auf Methylmethacrylat bzw. verwandter Substanzen, teilweise unter Zusatz weiterer Ester der Acryl- bzw. Methacrylsäure. Solche Knochenzemente sind z.B. in der DE 196 41 775 A1 beschrieben. Häufig wird dabei die Kombination Benzoylperoxid/Dimethyl-p-toluidin als Katalysator im flüssigen Monomer verwendet, wie in der DE 196 35 205 nachteilig herausgestellt. üblicherweise werden Knochenzemente aus zwei Komponenten angemischt. Eine Komponente enthält das flüssige Monomer, die andere Komponente ein pulverförmiges Polymerisat, das in Form kugelförmiger Partikel mit einem Durchmesser von ca. 100 *µ*m vorliegt.

Für die zur Kontrolle erforderliche Röntgenopazität werden Röntgenkontrastmittel zugesetzt. Bekannte Röntgenkontrastmittel sind BaSO₄ und ZrO₂, die in Mengen zwischen 7 und 30% zugesetzt werden.

Inzwischen sind eine Vielzahl von Knochenzementen in der Anwendung, dennoch sind sie noch immer mit Nachteilen behaftet.

Ein grundsätzliches Problem besteht darin, daß während der Polymerisation exotherm Wärme freigesetzt wird. Steigt die dabei auftretende Temperatur über 50°C an, werden die im Kontakt befindlichen Knochenzellen geschädigt. Die tatsächliche thermische Beanspruchung von Körperzellen an der Kontaktzone zum polymerisierenden Knochenzement ist nur mit großer Ungenauigkeit vorherzusagen. Sie hängt von der Dicke der eingebrachten Schicht und der Wärmeleitfähigkeit über die Prothesenkomponenten und vom Knochen ab. Laborversuche zeigten, daß unter bestimmten Bedingungen mit handelsüblichen Zementen während der Polymerisation Maximaltemperaturen von bis zu 110°C auftreten können, so daß Verbrennungen als Folgeerscheinungen zu beobachten sind. Hier erscheinen Verbesserungen notwendig.

Ein weiteres Problem der bisher bekannten Knochenzemente ist damit verbunden, daß sowohl stets vorhandenes restliches Monomer als auch andere Zusätze wie der Stabilisator Hydrochinon (Giftklasse 3) sowie der Akzelerator N,N-Dimethyl-p-toluidin (Giftklasse 2) herausgelöst und damit schädigend wirken können.

Weiterhin nachteilig kann sich das aus der Polymerisation ergebende Schrumpfen auswirken, was sich schließlich in der Lockerung der Prothese widerspiegeln kann.

Der Erfindung liegt die Aufgabe zugrunde, bisherige polymerisationsbedingte Komponenten oder Wirkungen zu vermeiden und gleichzeitig dem Knochenzement Langzeitstabilität, Bioaktivität und chemische Beständigkeit zu verleihen.

Das erfindungsgemäße Verfahren zur Herstellung eines Knochenzements löst die o.g. Probleme, indem die Polymerisation als solche bei der Bildung des Knochenzements vollständig vermieden wird. Erfindungsgemäß besteht das Verfahren darin, daß man
15 bis 50 Gew-% eines monomerfreien Polymethylmethacrylates (PMMA) mit einer mittleren Molmasse von 3000 bis 200000 Dalton und einer Säurezahl von 10 bis 350 mg KOH pro g Polymer mit einem biologisch verträglichen, organischen Lösungsmittel oder Lösungsmittelgemisch für das PMMA vermischt, und der Mischung 0,05 bis 80 Gew-% eines bioaktiven, glasig-kristallinen Materials mit einer Korngröße im Bereich von >20 bis 200 *µ*m unter Rühren und bei einer Temperatur von 10 bis 50°C zusetzt bis zum Erhalt einer fließfähigen Mischung mit einer offenen Verarbeitungszeit im Bereich von 1 bis 20 Minuten, wobei das glasig-kristalline Material aus 15 - 45 Gew-% CaO, 40 - 45 Gew-% P₂O₅, 10 - 40 Gew-% ZrO₂ und 0,7 - 3,5 Gew-% Fluorid besteht und als Hauptkristallphasen Apatit und Calciumzirconiumphosphat und als Nebenbestandteil eine Glasphase enthält, worin die Hauptkristallphasen zusammen wenigstens 35 Gew-% betragen und die Nebenbestandteile 5 bis 15 Gew-% betragen.

Durch den Verzicht auf eine innerhalb der Mischung ablaufende Polymerisationsreaktion kann ein Einsetzen und Aushärten bei Körpertemperatur realisiert werden. Zu diesem Zweck wird ein säurezahlmodifiziertes PMMA entsprechender Molmasse in einem dafür geeigneten Lösungsmittel gelöst, z.B. Acetylessigsäure-ethylester oder Mischungen von Acetylessigsäureethylester mit Ethanol, der bis zu 4 Vol-% Wasser enthalten kann. Die sich ergebende klebrige, fließende Komponente wird nun mit einem Pulvergemisch aus dem glasig-kristallinen Material und gegebenenfalls aus zusätzlicher vollständig oder teilweise resorbierbarer und/oder langzeitstabiler Biokeramik sowie gegebenenfalls TiO₂ vermengt. Dabei haben die pulverförmigen Bestandteile Korngrößen im Bereich von > 20 bis 200 *µ*m. Im Ergebnis dieser Prozedur entsteht ex vivo eine fließfähige, spritzbare und formbare Masse, die über einen Zeitraum von einigen Minuten, z.B. 1 - 10 min, in Abhängigkeit vom Pulveranteil, verarbeitet werden kann.

Bevorzugt wird ein Polymethylmethacrylat mit einem Anteil von 30 bis 35 Gew-% eingesetzt.

Die mittler Molmasse des PMMA kann vorteilhaft im Bereich von 20000 und 80000 Dalton liegen.

Die Säurezahl kann vorteilhaft im Bereich von 25 bis 65 mg KOH pro g Polymer liegen. Die Säurezahl gibt in diesem Zusammenhang die Anzahl der mg KOH an, die zur Neutralisation von 1 g der Polymerprobe verbraucht wird. Sie ist ein wesentliches Kriterium, da die Anzahl der freien Carboxylgruppen am Polymeren für die Bindung der Metallkomponenten wichtig ist.

Das säurezahlmodifizierte Acrylat kann hergestellt werden aus Methylmethacrylat und Methacrylsäure in einer Suspensionspolymerisation, wobei das Verhältnis der Molmasse so gewählt werden muß, daß die gewünschte Säurezahl erhalten wird. Desweiteren wird das säurezahlmodifizierte Polymere durch alkalische Verseifung eines Polymeren aus Methylmethacrylat und Ethylmethacrylat erhalten. Der Anteil des Ethylmethacrylates liegt zwischen 2 und 10 Mol, vorzugsweise bei 6 Mol.

Ein bevorzugtes glasig-kristallines Material enthält 23 - 39 Gew-% CaO, 40 - 45 Gew-% P₂O₅, 20 - 35 Gew-% ZrO₂ und 1-3 Gew-% Fluorid und enthält als Hauptkristallphasen Apatit und calciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen.

Ein ebenfalls bevorzugtes glasig-kristallines Material enthält 23 - 39 Gew-% CaO, 40 - 45 Gew-% P₂O₅, 20 - 35 Gew-% ZrO₂ und 1 - 3 Gew-% Fluorid sowie zusätzlich 0,1 bis 6 Gew-% Na₂O, und es enthält als Hauptkristallphasen Apatit und Calciumzirconiumphosphat und als Nebenbestandteil eine Glasphase und zusätzlich als Nebenbestandteil eine Natriumzirconiumphosphat-Phase. Dabei beträgt der Anteil der Hauptkristallphasen zusammen wenigstens 35 Gew-%, und die Nebenbestandteile können jeweils 5 bis 15 Gew-% betragen.

Weiterhin kann das erfindungsgemäße glasig-kristalline Material zusätzlich 0,1 bis 6 Gew-% Magnesiumoxid und/oder Kaliumoxid enthalten und zusätzlich auch die entsprechenden Phasen.

Der Gehalt an Na₂O, MgO und/oder K₂O liegt vorzugsweise im Bereich von 1 bis 6 Gew-%. Der Anteil der entsprechenden Nebenkristallphase Natriumzirconiumphosphat liegt vorzugsweise im Bereich von 5 bis 10 Gew-%.

Die Herstellung des glasig-kristallinen Materials erfolgt durch Gemengebildung mit geeignete Substanzen, also mit 15 - 45 Gew-% CaO, 40 - 45 Gew-% P₂O₅, 10 - 40 Gew-% ZrO₂ und 0,7- 3,5 Gew-% Fluorid.Das Fluorid wird vorteilhaft als CaF₂ eingebracht. Die Gemengebestandteile werden miteinander kombiniert und in geeigneten meist mehrstufigen Temperaturbehandlungsprogrammen (Haltestufen im Bereich von 400 bis 1500 °C) in einem geeigneten Tiegelmaterial, vorzugsweise bestehend aus einer Pt/Rh-Legierung, bei 1550 bis 1650 °C zum Schmelzen gebracht. Die Schmelze wird vergossen, und die erstarrte Schmelze wird je nach Verwendungszweck an der Luft (spontane Abkühlung) oder im Kühlofen auf Raumtemperatur abgekühlt. Danach wird der Werkstoff aufgemahlen.

Das so hergestellte glasig-kristalline Material ist ein solches, das einen oder mehrere der folgenden Parameter aufweist
- Gesamtlöslichkeit von 4 bis 5,5 mg/l, wenn die Prüfung in 0,2M TRIS-HCl-Puffer-Lösung bei pH=7,4, T=37 °C, an einer Kornfraktion von 315 - 400 *µ*m, 120h lang bei einem Probenoberflächen-zu-Lösungsmittelvolumen-Verhältnis von 5cm⁻¹ erfolgt,
- thermischer Ausdehnungskoeffizient zwischen 1,4 und 6•10⁻⁶ grad⁻¹ zwischen 27°C und 800°C,
- Stabilität im sauren pH-Bereich zwischen 7,0 und 7,5.

Die hier verwendeten Begriffe "Glaskeramik" und "glasig-kristallines Material" sind im Allgemeinen nicht immer eindeutig definierbar. Es liegen sowohl kristalline als auch glasige bzw. röntgenamorphe Phasen innig vermischt vor. Für die vorliegende Erfindung ist es ohne Belang, ob eine Phase neben der anderen vorliegt oder ob eine Phase die andere umhüllt.

Als "Hauptkristallphase" wird hier eine kristalline Phase bezeichnet, deren Mengenanteil wenigstens doppelt so groß ist, wie die einer Nebenphase, wobei Konzentrationen um 15% und darunter, vorzugsweise unter 10 Gew-% als Nebenphasen bezeichnet werden.

Als zusätzlich zu dem genannten glasig-kristallinen Material einsetzbares biokeramisches Material wird vorteilhaft ein Material ausgewählt, das Natrium, Kalium, calcium, Magnesium, Hydroxyl-Ionen oder Hydroxyl-Bestandteile, Fluorid, Silicat und/oder ortho-Phosphat enthält. Bevorzugt ist ein biokeramisches Material ein solches mit kristallinen Phasen von Ca₂KNa(PO₄)₂ und einer inneren offenen Porenstruktur. Der Zusatz von resorbierbaren Biokeramiken bietet die Möglichkeit des Aufbaus poröser Strukturen, die osteokonduktiv und gleichzeitig stützend wirken können. Der Prozeß der Auflösung der Biokeramikpartikelchen ist abhängig von deren Struktur und kann nach Wunsch eingestellt werden. Als vorteilhaft hat sich u.a. ein Material erwiesen, daß gemäß DE 19744809 C1 hergestellt wurde bzw. Werkstoffe, die Ca₂KNa(PO₄)₂ oder ähnliche Phasen enthalten. Verwendet man hingegen langzeitstabile, bioaktive Keramiken oder Glaskeramiken, so sollte eine der kristallinen Phasen Apatit sein. Als vorteilhaft hat sich eine Glaskeramik auf der Basis Apatit/Wollastonit gemäß DD 247574A3 erwiesen.

Die Teilchengröße (Korngröße, Körnung) kann vorzugsweise im Bereich von 25 bis 160 *µ*m liegen, insbesondere bei 25 bis 90 *µ*m. Die Messung der Teilchengröße erfolgt mit der Lasergranulometrie.

Zur Erzielung eines röntgendichteren Werkstoffes empfiehlt es sich, ein Material dem erfindungsgemäß herzustellenden Knochenzement-Komposit beizumischen, das aus folgenden Komponenten besteht bzw. sie in Anteilen größer 30 Masse-% enthält, nämlich: CaZr₄(PO₄)₆ und/oder CaTi₄(PO₄)₆. Für den vorliegenden Anwendungsfall ist es von der Sache her gleichgültig, ob Calcium-Zirkonium- und/oder Calcium-Titan-Orthophosphat in amorpher oder der eher typischen kristallinen Form vorliegt.

Es wurde weiterhin gefunden, daß als zusätzlicher anorganischer Füllstoff TiO₂ vorzugsweise in Mengen von 0,1 bis 10 Gew-%, bezogen auf das Gesamtgewicht des Zements, und bevorzugt in der Modifikation Rutil zugegeben werden kann und daß dadurch wesentlich erhöhte Festigkeiten erzielt werden können.

Auf Grund seiner Struktur weist der Zement zudem eine Klebrigkeit gegenüber Metalloxiden auf, mit der Folge einer verbesserten Haftung an z.B. keramischen Oberflächen oder Implantaten aus Titanlegierungen mit der jeweils vorgelagerten Oxidschicht.

In das erfindungsgemäße Verfahren miteinbezogen werden können Arzneimittel, wie z.B. Antibiotika, die vorteilhaft einzelnen Mischungskomponenten, z.B. dem biokeramischen Material, zugesetzt werden können oder auch allein in das Gemisch eingebracht werden. Bevorzugt ist Gentamycin mit einem Anteil von etwa 0,5 bis 2 Gew-%, vorzugsweise 0,8 bis 1,3 Gew-%, bezogen auf die Gesamtmasse des Zements.

Ein besonderer Vorteil des erfindungsgemäßen Zements besteht darin, daß es sich hierbei um einen zink- und monomerfreien Zement handelt, der einfach anmischbar ist, in seiner Thixotropie und/oder Porengröße einstellbar ist und keine toxischen Substanzen an die Umgebung abgibt. Der erfindungsgemäße Zement ist zinkfrei, was besonders vorteilhaft ist, da Zink in höheren Konzentrationen toxisch wirken kann (Contzen et al., Grundlagen der Alloplastik mit Metallen und Kunststoff, Thieme Verlag Stuttgart, 1967, S. 56). Insbesondere die fehlende Toxizität infolge der Vermeidung von Zink und Monomeren sowie üblichen Stabilisatoren und Akzeleratoren ist hervorzuheben. Weiterhin vorteilhaft ist, daß er auch nicht während des Anrührvorgangs zwischen 1 und 10 Minuten, vorzugsweise 4-5 min, aushärtet, und somit eine plastische Phase von durchschnittlich 3 bis 8 min gegeben ist.

Alle diese Faktoren führen zu einer guten Benetzung des Zements auf der Implantatoberfläche wie auch des Knochen und somit zu einer gleichmäßigen Schichtdicke. Damit kann ein gleichmäßiger Kontakt zwischen Implantat und Knochen vermittelt durch den Zement gewährleistet werden. Verarbeitungsfehler treten dadurch wesentlich vermindert auf.

Ein weiterer Vorteil ist die Form- und Volumenbeständigkeit des erfindungsgemäßen Knochenzements, bei dem Schrumpfvorgänge wesentlich reduziert sein können. Eine Optimierung führt zu Ergebnissen deutlich unter 1 %.

Wesentlicher Vorteil des erfindungsgemäßen Verfahrens ist ferner, daß durch Vermeidung der herkömmlichen Polymerisationsreaktion die sonst stets auftretende Temperaturerhöhung der exothermen Reaktion und damit die Schädigung umliegender Zellen durch Temperaturen größer etwa 50-60 °C vollständig vermieden wird (zu den Nachteilen derartiger Polymerisationsreaktionen siehe Liebergall et al., Clin.Orthop. 1998 Apr. (349)242-248 und Sturup et al., Acta Orthop. Scand. 1994 Feb. 65(1), 20-23).

Mit Hilfe des Molekulargewichtes des PMMA und der Anzahl der funktionellen Gruppen (Säurezahl) kann die Porengröße ebenfalls eingestellt werden, z.B. können Poren im Bereich von 1*µ*m bis 159*µ*m erreicht werden.

Durch Einsatz des bioaktiven glasig-kristallinen Material sowie gegebenenfalls weiterer biokeramischer Pulver können für des Einsprießen von Zellen optimale Hohlräume infolge des Auflösens von Pulverteilchen geschaffen werden.

Der Aushärtevorgang wird durch die Ausbildung von Chelatverbindungen hervorgerufen. Diese können durch teilweise lösliche Bestandteile der Keramiken gebildet werden.

Weiterhin kann das Verfahren vorteilhaft gestaltet werden, indem die Porosität des ausgehärteten Zements über einen Anteil an resorbierbarem biokeramischen Material eingestellt wird, der im Bereich von 5 bis 80 Gew-%, vorzugsweise 10 bis 40 Gew-% liegen kann, bezogen auf das Gesamtgewicht des Knochenzements. Die Viskosität des form- und spritzbaren Zements wird über den Anteil der Mischungskomponenten und/oder das Molekulargewicht des PMMA eingestellt.

Die Stabilität, charakterisiert durch den Elastizitätsmodul (aus Biegefestigkeitsmessungen ermittelt), ist durch das Verhältnis von langzeitstabilem glasig-kristallinem bzw. resorbierbarem anorganischen Material und gelöstem Polymer in einem Bereich von 5 bis 50 MPa einstellbar.

Die Erfindung betrifft ferner einen Knochenzement-Kit auf Basis von Polymethylmethacrylat, gekennzeichnet durch die folgenden, getrennt voneinander vorliegenden Bestandteile
a) 15 bis 50 Gew-% eines monomerfreien Polymethylmethacrylates (PMMA) mit einer mittleren Molmasse von 3000 bis 200000 Dalton und einer Säurezahl von 10 bis 350 mg KOH pro g Polymer;
b) 5 bis 40 Gew-% eines biologisch verträglichen, organischen Lösungsmittels oder Lösungsmittelgemisches für das PMMA;
c) 0,05 bis 80 Gew-% eines bioaktiven, glasig-kristallinen Materials mit einer Korngröße im Bereich von >20 bis 200 *µ*m, wobei das glasig-kristalline Material aus 15 - 45 Gew-% CaO, 40 - 45 Gew-% P₂O₅, 10 - 40 Gew-% ZrO₂ und 0,7 - 3,5 Gew-% Fluorid besteht und als Hauptkristallphasen Apatit und Calciumzirconiumphosphat und als Nebenbestandteil eine Glasphase enthält, worin die Hauptkristallphasen zusammen wenigstens 35 Gew-% betragen und die Nebenbestandteile 5 bis 15 Gew-% betragen.

Der Knochenzement-Kit kann zusätzlich einzeln oder als Gemisch mit der Komponente c) Bestandteile enthalten, die ausgewählt sind aus der Gruppe, bestehend aus TiO₂, Röntgenkontrastmittel wie CaZr₄(PO₄)₆ oder CaTi₄(PO₄)₆, ein resorbierbares biokeramisches Material mit kristallinen Phasen von Ca₂KNa(PO₄)₆ und einer inneren offenen Porenstruktur, eine langzeitstabile Glaskeramik auf der Basis Apatit/Wollastonit (gemäß DD 247574) oder Gemische davon.

Das biologisch verträgliche Lösungsmittel, das zu dem erfindungsgemäßen Knochenzement-Kit gehört, ist Acetessigsäureethylester oder ein Gemisch von Acetessigsäureethylester mit Ethanol, wobei Ethanol bis zu 4 Vol-% Wasser enthalten kann. Vorzugsweise ist es Acetessigsäureethylester.

Der erfindungsgemäße Kit liegt in sterilisierter Form vor, wobei die Sterilisation durch Ethylenoxid oder mittels Strahlensterilisation vorgenommen werden kann.

Weiterhin kann der Kit Arzneimittelkomponenten im Gemisch mit den Einzelkomponenten oder gesondert enthalten, insbesondere Antibiotika.

Die Erfindung soll nachstehend durch Beispiele näher erläutert werden. Alle Prozentangaben sind auf das Gewicht bezogen.

### Beispiel 1 Herstellung des glasig-kristallinen Materials Apatit/CZP1

Es wird ein Gemenge bereitet, das folgender Zusammensetzung entspricht (Code: Apatit/CZP1):
25,88 CaO
28,44 ZrO₂
43,68 P₂O₅
5,00 CaF₂
Dabei erweist es sich als praktikabel, den CaO-Anteil in Form von 62,79 CaHPO₄ einzusetzen und den noch notwendigen P₂O₅-Anteil in Form von 10,51ml einer 85%igen H₃PO₄. Zunächst wird CaHPO₄, ZrO₂ und CaF₂ gut vermischt, danach mit der Phosphorsäure versetzt, nach Reaktion gemörsert, wobei 4h Trockenhaltestufen von 120°C und 170°C eingelegt wurden. Diese Reaktionsgemisch wird in einem Pt/Rh-Tiegel gefüllt und über die 1h Haltestufen 400 und 800°C erhitzt, abgekühlt und anschließend gemörsert. Das so vorbehandelte Material wird nunmehr im Pt/Rh-Tiegel mit jeweils 15min Haltezeit in den Stufen 800, 1000, 1300, 1500 und schließlich 1600°C zum Schmelzen gebracht und sodann auf eine Stahlplatte (Raumtemperatur) gegossen.

Ein Teil der Schmelze wurde durch Mahlen in einer Achatmühle zerkleinert, unter 43*µ*m abgesiebt und sodann einer röntgendiffraktographischen Untersuchung unterzogen. Das Ergebnis im Röntgendiffraktogramm zeigt, daß in dem glasig-kristallinen Produkt die Kristallphasen Apatit (Fluorapatit/Hydroxylapatit) und Calciumzirconiumphosphat [CaZr₄(PO₄)₆] deutlich nachweisbar sind. Der restliche Teil der Schmelze wird auf eine Körnung von >20 bis 200 *µ*m gebracht.

### Beispiel 2 Herstellung des glasig-kristallinen Materials Apatit/CZP2

Es wird ein Gemenge nach der Vorschrift des Beispiels 1 hergestellt mit dem Unterschied, daß hier als zusätzliche Komponente Natriumoxid eingebracht wird (Code: Apatit/CZP2). Der Ansatz sieht die Verwendung folgender Gemengebestandteile vor:
59,93 CaHPO₄
27,10 ZrO₂
3,42 Na₂O
5,00 CaF₂ und
9,56ml einer 85%igen H₃PO₄-Säure.
Es wurde wie im Beispiel 1 gearbeitet. Nach der letzten Temperaturhaltestufe wurde die Schmelze aus dem Tiegel auf eine Stahlplatte gegossen.
Ein Teil der Schmelze wurde durch Mahlen in einer Achatmühle zerkleinert, unter 43*µ*m abgesiebt und sodann einer röntgendiffraktographischen Untersuchung unterzogen. Das Ergebnis im Röntgendiffraktogramm zeigt, das in dem glasig-kristallinen Produkt die Kristallphasen Apatit (Fluorapatit/Hydroxylapatit) und Calciumzirconiumphosphat [CaZr₄(PO₄)₆] und Natriumzirconiumphosphat [NaZr₂(PO₄)₃] nachweisbar sind.
Der restliche Teil der Schmelze wird auf eine Körnung von >20 bis 200 *µ*m gebracht.

### Beispiel 3 Ausdehnungskoeffizienten Apatit/CZP1

Es erfolgte die Herstellung eines glasig-kristallinen Materials nach Beispiel 1 (Apatit/CZP1). Das Material wird durch Mahlen in einer mit Zirconiumoxid ausgekleideten Mühle zerkleinert, so daß sich ein D₅₀-Wert von 8*µ*m ergab. Das gemahlene Gut wird mit einer 5%igen Polyvinylalkohol(PVA)-Lösung im Verhältnis Mahlgut zu PVA-Lösung von 90 : 10 Masse-% versetzt und zu einem Stab mit 4,7kN in einem Preßgesenk verpreßt. Dieser Rohkörper wird gesintert bei einer Temperatur von 1050°C.

An dem auf diese Weise erhaltenen relativ dichten Formkörper wird der thermische Ausdehnungskoeffizient(AK) bestimmt:
AK im Bereich von 27 - 400°C: 1,90*10⁻⁶ grd Celsius⁻¹
AK im Bereich von 50 - 400°C: 1,86*10⁻⁶ grd Celsius⁻¹
AK im Bereich von 30 - 300°C: 1,45*10⁻⁶ grd Celsius⁻¹
AK im Bereich von 30 - 400°C: 1,88*10⁻⁶ grd Celsius⁻¹
AK im Bereich von 30 - 600°C: 2,6*10⁻⁶ grd Celsius⁻¹
AK im Bereich von 30 - 800°C: 3,2*10⁻⁶ grd Celsius⁻¹

### Beispiel 4 Chemische Beständigkeit Apatit/CZP1 im basischen Bereich

Es erfolgt die Herstellung eines glasig-kristallinen Materials nach Beispiel 1 (Apatit/CZP1). Sodann wird das Material gemörsert und eine Kornfraktion von 315 - 400*µ*m daraus hergestellt.
Das auf diese Weise erhaltene Granulat wird im Vergleich zu einem Grundglas (Ap40_{Glas}) und einer aus diesem Grundglas hergestellten Glaskeramik auf der Basis von Apatit und Wollastonit (Ap40ₖᵣᵢₛₜ.) [chemische Zusammensetzung also identisch zu (Gewichts-%): 44,3 SiO₂; 11,3 P₂O₅; 31,9 CaO; 4,6 Na₂O; 0,19 K₂O; 2,82 MgO und 4,99 CaF₂] hinsichtlich seiner chemischen Beständigkeit verglichen.

Zu diesem Zweck wurden zunächst die spezifischen Oberflächen nach BET mit Krypton als Meßgas bestimmt zu:
Apatit/CZP1: 0,364m²/g
Ap⁴⁰_{Glas} : 0,018 m²/g
Ap40ₖᵣᵢₛₜ : 0,055m²/g.
Hier zeigt sich, daß das im erfindungsgemäßen Knochenzement eingesetzte glasig-kristalline Material eine gewisse offene Porosität im Vergleich zum Grundglas und der daraus hergestellten Glaskeramik aufweist. Diesen Unterschieden wird bei den Lösungsuntersuchungen dadurch Rechnung getragen, indem das Oberflächen(Proben) zu Lösungsmittelvolumen(TRIS-HCl-Puffer-Lösung) konstant auf 5cm⁻¹ eingestellt wird.
Als Lösungsmittel wurde eine 0,2M TRIS-HCl-Puffer-Lösung mit pH=7,4 bei 37°C verwendet. Die Lagerung erfolgte bei 37°C über eine Zeitdauer von 120h. Danach wurde die Gesamtlöslichkeit durch Bestimmung der Einzelionen (Ca, P, Zr) in der Lösung mit Hilfe einer ICP-Messung bestimmt zu:
Apatit/CZP1: 4,1 - 5,1 mg/L
Ap40_{Glas}: 318 - 320mg/L
Ap40_{krist.}: 75,2 - 82,0 mg/L.
Die Werte belegen eindrucksvoll die hohe chemische Beständigkeit des in dem erfindungsgemäßen Knochenzement eingesetzten neuen Werkstoffes unter simulierten physiologischen Bedingungen, einer bekannten Methode zur Bestimmung der Langzeitbeständigkeit in vitro.

### Beispiel 5 Chemische Beständigkeit Apatit/CZP1 im sauren Bereich

Es wird vorgegangen wie im Beispiel 4, jedoch wird eine 0,2M TRIS-HCl-Puffer-Lösung mit dem pH-Wert von 6,0 bei 37°C zur Messung verwendet. Auf diese Weise läßt sich der Fall simulieren, daß infolge einer Wundheilungs- oder Spätinfektion der pH-Wert von den physiologischen 7,4 in den sauren Bereich abgleitet.
Mit Hilfe der ICP wurden nun folgende Werte der Gesamtlösung bestimmt:
Apatit/CZP1: 16-19 mg/L
Ap40_{Glas}: 505-518 mg/L
Ap40ₖᵣᵢₛₜ : 117-125 mg/L.
Die Werte belegen eindrucksvoll die hohe chemische Beständigkeit des für die Erfindung eingesetzten Werkstoffes unter simulierten Bedingungen, wie sie bei einer Entzündungsreaktion vorliegen. Demzufolge ist die Erhöhung der absoluten Werte der Löslichkeit für den erfindungsgemäßen Werkstoff wesentlich geringer im Vergleich zu der doch dramatischen Erhöhung im Falle des Grundglases bzw. der Glaskeramik auf Apatit/Wollastonit-Basis.

### Beispiel 6 Herstellung I des Knochenzements

Ausgangsmaterial war ein monomerfreies, säurezahlmodifiziertes Polymethylmethacrylat (PMMA) mit einer mittleren Molmasse von ca. 100000. 3g des PMMA (Säurezahl 62mg KOH/g) wurden in 7g einer Mischung aus 50 Teilen Ethanol (abs.) und 60 Teilen Acetetessigsäureethylester in eine 30 Gew-% Lösung unter Rühren überführt. Danach wurde ein Gemisch aus glasig-kristallinem Material bzw. biokeramischem Material unter Rühren bis zur Homogenität bei Raumtemperatur (18-25 °C) eingearbeitet.
Das erhaltene Gesamtgemisch mit cremiger Konsistenz wurde als Knochenzement innerhalb einer der jeweiligen Abbindezeit verarbeitet.
In der nachfolgenden Tabelle sind die jeweiligen Angaben für die Einzelkomponenten als prozentuale Anteile des Gesamtgemisches angegeben. Polymeransatz bedeutet Polymeres + Lösungsmittel.

Die zugesetzten Biokeramiken bzw. das glasig-kristalline Material hatten eine mittlere Korngröße von 50 - 200 *µ*m. Folgende Materialien wurden eingesetzt:

| | | |
|---|---|---|
| Resorbierbare Biokeramik (DE 19744809) | 56-90 *µ*m | 21 Gew-% |
| Apatit/CZP2 (Beispiel 2) | 71 - 100 *µ*m | 21 Gew-% |
| Tetracalciumphosphat | 20 *µ*m | 21 Gew-% |
| TiO₂ | | 10,4 Gew-% |

Das Material ließ sich sehr leicht anrühren und hatte eine klebrig-cremige Konsistenz. Es war spritzbar und wasserbeständig. Man erreichte Porendurchmesser bis 150 *µ*m. Die Biegefestigkeit betrug 12,2 MPa.

### Beispiel 7 Herstellung II des Knochenzements

Ausgangsmaterial war ein monomerfreies, säurezahlmodifiziertes Polymethylmethacrylat (PMMA) mit einer mittleren Molmasse von ca. 100000. 3g des PMMA (Säurezahl 62mg KOH/g) wurden in 7g einer Mischung aus 60 Teilen Ethanol (96%) und 50 Teilen Acetessigsäureethylester in eine 30 Gew-% Lösung unter Rühren überführt. Danach wurde ein Gemisch aus biokeramischem Material unter Rühren bis zur Homogenität bei Raumtemperatur (18-25°C) eingearbeitet.
Das erhaltene Gesamtgemisch mit cremiger Konsistenz wurde als Knochenzement innerhalb einer der jeweiligen Abbindezeit verarbeitet.
In der nachfolgenden Tabelle sind die jeweiligen Angaben für die Einzelkomponenten als prozentuale Anteile des Gesamtgemisches angegeben.
Die zugesetzten Biokeramiken hatten eine mittlere Korngröße von 50 - 200 *µ*m. Folgende Biokeramiken wurden eingesetzt:

| | | |
|---|---|---|
| Resorbierbare Biokeramik (DE 19744809) | 56 - 90*µ*m | 21 Gew-% |
| Apatit/CZP2 (Material von Beispiel 2) | 71 - 100 *µ*m | 21 Gew-% |
| Tetracalciumphosphat | 20 *µ*m | 21 Gew-% |
| TiO₂ | | 10,4 Gew-% |

Das Material ließ sich leicht anrühren und hatte eine klebrig-cremige Konsistenz. Es war spritzbar und wasserbeständig.
Man erreichte Porendurchmesser bis 150 *µ*m. Die Biegefestigkeit betrug 10,4 MPa.

## Patentansprüche

1. Verfahren zur Herstellung eines bioaktiven Knochenzements, **dadurch gekennzeichnet, daß** man
15 bis 50 Gew-%, bezogen auf das Gesamtgewicht des Knochenzements, eines monomerfreien Polymethylmethacrylates (PMMA) mit einer mittleren Molmasse von 3000 bis 200000 Dalton und einer Säurezahl von 10 bis 350 mg KOH pro g Polymer
mit einem biologisch verträglichen, organischen Lösungsmittel oder Lösungsmittelgemisch für das PMMA vermischt, und
der Mischung 0,05 bis 80 Gew-%, bezogen auf das Gesamtgewicht des Knochenzements, eines bioaktiven, glasig-kristallinen Materials mit einer Korngröße im Bereich von >20 bis 200 *µ*m zusetzt, bestehend aus 15 - 45 Gew-% CaO, 40 - 45 Gew-% P₂O₅, 10 - 40 Gew-% ZrO₂ und 0,7 - 3,5 Gew-% Fluorid, bezogen auf das Gesamtgewicht des glasig-kristallinen Materials, und als Hauptkristallphasen Apatit und Calciumzirconiumphosphat und als Nebenbestandteil eine Glasphase aufweisend, worin die Hauptkristallphasen des glasig-kristallinen Materials zusammen wenigstens 35 Gew-% betragen und die Nebenbestandteile 5 bis 15 Gew-% betragen,
unter Rühren und bei einer Temperatur von 10 bis 50 °C bis zum Erhalt einer fließfähigen Mischung mit einer offenen Verarbeitungszeit im Bereich von 1 bis 20 Minuten.

2. Verfahren nach Anspruch 1, **dadurch gekennzeichnet, daß** ein Polymethylmethacrylat mit einem Anteil von 30 bis 35 Gew-% eingesetzt wird.

3. Verfahren nach Anspruch 1, **dadurch gekennzeichnet, daß** als biologisch verträgliches Lösungsmittel Acetessigsäureethylester oder ein Gemisch von Acetessigsäureethylester mit Ethanol, wobei Ethanol bis zu 4 Vol-% Wasser enthalten kann, eingesetzt wird, vorzugsweise Acetessigsäureethylester.

4. Verfahren nach Anspruch 1, **dadurch gekennzeichnet, daß** dem glasig-kristallinen Material ein weiteres biokeramisches Material zugesetzt wird, insbesondere eine langzeitstabile oder vollständig oder teilweise resorbierbare Biokeramik.

5. Verfahren nach Anspruch 4, **dadurch gekennzeichnet, daß** als biokeramisches Material ein solches mit kristallinen Phasen von Ca₂KNa(PO₄)₂ ohne oder mit durchgehend offener Porenstruktur eingesetzt wird oder eine langzeitstabile Glaskeramik auf der Basis von Apatit/Wollastonit.

6. Verfahren nach Anspruch 1, **dadurch gekennzeichnet, daß** als glasig-kristallines Material ein solches mit der Zusammensetzung 23 - 39 Gew-% CaO, 40 - 45 Gew-% P₂O₅, 20 - 35 Gew-% ZrO₂ und 1 - 3 Gew-% Fluorid eingesetzt wird.

7. Verfahren nach Anspruch 1, **dadurch gekennzeichnet, daß** als glasig-kristallines Material ein solches eingesetzt wird, das zusätzlich 0,1 bis 6 Gew-% Na₂O enthält und zusätzlich als Nebenbestandteil eine Natriumzirconiumphosphat-Phase enthält.

8. Verfahren nach Anspruch 1, **dadurch gekennzeichnet, daß** den Ausgangskomponenten oder Gemischen davon ein Arzneimittel zugesetzt wird.

9. Verfahren nach Anspruch 1, **dadurch gekennzeichnet, daß** als weiterer anorganischer Füllstoff TiO₂ zugegeben wird, vorzugsweise 0,1 bis 10 Gew-% und weiterhin bevorzugt in der Modifikation Rutil.

10. Verfahren nach Anspruch 1, **dadurch gekennzeichnet, daß** zur Erzielung eines röntgendichten Zements bis zu 30 Gew-% CaZr₄(PO₄)₆ oder CaTi₄(PO₄)₆ oder Gemische davon oder Mischkristalle daraus zugegeben werden.

11. Verfahren nach einem der Ansprüche 1 bis 10, **dadurch gekennzeichnet, daß** die Porosität des ausgehärteten Zements über den Anteil an resorbierbarem biokeramischen Material eingestellt wird.

12. Verfahren nach einem der Ansprüche 1 bis 11, **dadurch gekennzeichnet, daß** die Viskosität des form- und spritzbaren Zements über den Anteil der Mischungskomponenten und/oder das Molekulargewicht des PMMA eingestellt wird.

13. Knochenzement-Kit auf Basis von Polymethylmethacrylat, **gekennzeichnet durch** die folgenden, getrennt voneinander vorliegenden Bestandteile
a) 15 bis 50 Gew-%, bezogen auf das Gesamtgewicht des Knochenzements, eines monomerfreien Polymethylmethacrylates (PMMA) mit einer mittleren Molmasse von 3000 bis 200000 Dalton und einer Säurezahl von 10 bis 350 mg KOH pro g Polymer;
b) 10 bis 40 Gew-%, bezogen auf das Gesamtgewicht des Knochenzements, eines biologisch verträglichen, organischen Lösungsmittels oder Lösungsmittelgemisches für das PMMA;
c) 0,05 bis 80 Gew-%, bezogen auf das Gesamtgewicht des Knochenzements, eines bioaktiven, glasig-kristallinen Materials mit einer Korngröße im Bereich von > 20 bis 200 µm bestehend aus 15 - 4.5 Gew-% CaO, 40 - 45 Gew-% P₂O₅, 10 - 40 Gew-% ZrO₂ und 0,7 - 3,5 Gew-% Fluorid, bezogen auf das Gesamtgewicht des glasig-kristallinen Materials, und als Hauptkristallphasen Apatit und calciumzirconiumphosphat und als Nebenbestandteil eine Glasphase aufweisend, worin die Hauptkristallphasen des glasig-kristallinen Materials zusammen wenigstens 35 Gew-% betragen und die Nebenbestandteile 5 bis 15 Gew-% betragen.

14. Knochenzement-Kit nach Anspruch 13, **dadurch gekennzeichnet, daß** er zusätzlich einen Bestandteil enthält, ausgewählt aus der Gruppe, bestehend aus TiO₂, Röntgenkontrastmittel wie
CaZr₄ (PO₄) ₆ oder CaTi₄(PO₄)₆, ein resorbierbares biokeramisches Material mit kristallinen Phasen von Ca₂KNa(PO₄)₂ und einer inneren offenen Porenstruktur, eine langzeitstabile Glaskeramik auf der Basis Apatit/Wollastonit oder Gemische davon.

15. Knochenzement-Kit nach Anspruch 13, **dadurch gekennzeichnet, daß** das biologisch verträgliche Lösungsmittel Acetessigsäureethylester oder ein Gemisch von Acetessigsäureethylester mit Ethanol ist, wobei Ethanol bis zu 4 Vol-% Wasser enthalten kann, vorzugsweise Acetessigsäureethylester.

## Claims

1. A method for the production of a bioactive bone cement comprising the steps of
mixing 15 to 50% by weight, relative to the total weight of the bone cement, of a monomer-free polymethylmethacrylate (PMMA) whose average molar mass ranges between 3,000 and 200,000 daltons and whose acid value ranges between 10 and 350 mg KOH per g polymer
with a biocompatible, organic solvent or solvent mixture for the PMMA, and
adding to the mixture 0.05 to 80% by weight, relative to the total weight of the bone cement, of a bioactive vitreous-crystalline material with a particle size ranging between >20 and 200 µm, which material consists of 15-45% by weight CaO, 40-45% by weight P₂O₅, 10-40% by weight ZrO₂ and 0.7-3.5% by weight fluoride, relative to the total weight of the vitreous-crystalline material, and comprises apatite and calcium zirconium phosphate as main crystal phases and a glass phase as an auxiliary component, said main crystal phases of the vitreous-crystalline material jointly making up at least 35% by weight and said auxiliary components making up 5 to 15% by weight,
while stirring and at a temperature ranging between 10 and 50°C until a flowable mixture is obtained whose open processing time ranges between 1 and 20 minutes.

2. A method according to Claim 1 wherein a polymethylmethacrylate is used in an amount ranging between 30 and 35% by weight.

3. A method according to Claim 1 wherein the biocompatible solvent used is ethyl acetoacetate or a mixture of ethyl acetoacetate with ethanol, which ethanol may contain water up to an amount of 4% by volume, preferably ethyl acetoacetate.

4. A method according to Claim 1 wherein another bioceramic material, in particular a long-term stable or a totally or partially resorbable bioceramic, is added to the vitreous-crystalline material.

5. A method according to Claim 4 wherein the bioceramic material used is a material comprising crystalline phases of Ca₂KNa(PO₄)₂ with or without an open-pore structure throughout or a long-term stable glass ceramic based on apatite/wollastonite.

6. A method according to Claim 1 wherein the vitreous-crystalline material used is a material composed of 23-39% by weight CaO, 40-45% by weight P₂O₅, 20-35% by weight ZrO₂ and 1-3% by weight fluoride.

7. A method according to Claim 1 wherein the vitreous-crystalline material used is a material additionally containing 0.1 to 6% by weight Na₂O and additionally containing a sodium zirconium phosphate phase as an auxiliary component.

8. A method according to Claim 1 wherein a medicine is added to the starting components or mixtures thereof.

9. A method according to Claim 1 wherein TiO₂ is added as an additional inorganic filler, preferably in an amount ranging between 0.1 and 10% by weight and preferably in the form of its modification rutile.

10. A method according to Claim 1 wherein CaZr₄(PO₄)₆ or CaTi₄(PO₄)₆ or mixtures thereof or mixed crystals therefrom is/are added in an amount ranging up to 30% by weight in order to obtain an X-ray dense cement.

11. A method according to any of Claims 1 through 10 wherein the porosity of the hardened cement is adjusted by varying the amount of resorbable bioceramic material.

12. A method according to any of Claims 1 through 11 wherein the viscosity of the mouldable and sprayable cement is adjusted by varying the amount of the components making up the mixture and/or the molecular weight of the PMMA.

13. A bone cement kit based on polymethylmethacrylate comprising the following components provided separate of one another:
a) 15 to 50% by weight, relative to the total weight of the bone cement, of a monomer-free polymethylmethacrylate (PMMA) whose average molar mass ranges between 3,000 and 200,000 daltons and whose acid value ranges between 10 and 350 mg KOH per g polymer;
b) 10 to 40% by weight, relative to the total weight of the bone cement, of a biocompatible organic solvent or solvent mixture for the PMMA;
c) 0.05 to 80% by weight, relative to the total weight of the bone cement, of a bioactive, vitreous-crystalline material with a particle size ranging between >20 and 200 µm, which material consists of 15-45% by weight CaO, 40-45% by weight P₂O₅, 10-40% by weight ZrO₂ and 0.7-3.5% by weight CaF₂, relative to the total weight of the vitreous-crystalline material, and comprises apatite and calcium zirconium phosphate as main crystal phases and a glass phase as an auxiliary component, said main crystal phases of the vitreous-crystalline material jointly making up at least 35% by weight and said auxiliary components making up 5 to 15% by weight.

14. A bone cement kit according to Claim 13 wherein said kit additionally comprises a component selected from the group consisting of TiO₂, X-ray contrast media such as CaZr₄(PO₄)₆ or CaTi₄(PO₄)₆, a resorbable bioceramic material comprising crystalline phases of Ca₂KNa(PO₄)₆, and an internal open-pore structure, a long-term stable glass ceramic based on apatite/wollastonite and mixtures thereof.

15. A bone cement kit according to Claim 13 wherein the biocompatible solvent used is ethyl acetoacetate or a mixture of ethyl acetoacetate with ethanol, which ethanol may contain water up to an amount of 4% by volume, preferably ethyl acetoacetate.

## Revendications

1. Procédé de fabrication d'un ciment à os bioactif, **caractérisé en ce qu'**on mélange
15 à 50 % en masse, par rapport au poids total du ciment à os, d'un polyméthacrylate de méthyle (PMMA) exempt de monomère avec une masse moléculaire moyenne de 3000 à 200000 Dalton et un indice d'acidité de 10 à 350 mg KOH par g de polymère
à un solvant ou un mélange de solvant organique, biologiquement compatible pour le PMMA, et
ont été ajoutés au mélange 0,05 à 80 % en masse, par rapport au poids total du ciment à os, d'un matériau bioactif, vitro-cristallin avec une grosseur de grain dans la plage de >20 à 200 µm, constitué de 15 à 45 % en masse de CaO, 40 à 45 % en masse de P₂O₅, 10 à 40 % en masse de ZrO₂ et 0,7 à 3,5 % en masse de fluorure, par rapport au poids total du matériau vitro-cristallin, et comprenant comme phases cristallines principales de l'apatite et du phosphate de zirconium et calcium et comme composant minoritaire une phase vitreuse, dans lesquelles les phases cristallines principales du matériau vitro-cristallin sont conjointement d'au moins 35 % en masse et les composants minoritaires de 5 à 15 % en masse,
par malaxage et à une température de 10 à 50°C jusqu'à l'obtention d'un mélange coulant avec une durée libre de traitement dans la plage de 1 à 20 minutes.

2. Procédé selon la revendication 1, **caractérisé en ce qu'**on utilise avec une part de 30 à 35 % en masse un polyméthacrylate de méthyle.

3. Procédé selon la revendication 1, **caractérisé en ce qu'**on utilise de l'ester acétylacétique comme solvant biologiquement compatible ou un mélange d'ester acétylacétique à de l'éthanol, l'éthanol pouvant contenir jusqu'à 4 % en volume d'eau, de préférence de l'ester acétylacétique.

4. Procédé selon la revendication 1, **caractérisé en ce qu'**un autre matériau biocéramique est ajouté au matériau vitro-cristallin, en particulier une biocéramique stable à long terme ou totalement ou partiellement résorbable.

5. Procédé selon la revendication 4, **caractérisé en ce qu'**un matériau comprenant des phases cristallines de Ca₂Kna(PO₄)₂ sans ou avec structure poreuse ouverte en continu est utilisé comme matériau biocéramique ou une vitrocéramique stable à long terme à base d'apatite/de wollastonite.

6. Procédé selon la revendication 1, **caractérisé en ce qu'**un matériau comprenant la composition 23 à 39 % en masse de CaO, 40 à 45 % en masse de P₂O₅, 20 à 35 % en masse de ZrO₂ et 1 à 3 % en masse de fluorure est utilisé comme matériau vitro-cristallin.

7. Procédé selon la revendication 1, **caractérisé en ce qu'**un matériau qui contient en plus 0,1 à 6 % en masse de Na₂O et contient en outre comme composant minoritaire une phase de phosphate de zirconium et sodium est utilisé comme matériau vitro-cristallin.

8. Procédé selon la revendication 1, **caractérisé en ce qu'**un médicament est ajouté aux éléments constitutifs de départ ou à des mélanges de ceux-ci.

9. Procédé selon la revendication 1, **caractérisé en ce que** du TiO₂ est ajouté comme autre charge anorganique, de préférence 0,1 à 10 % en masse et en outre du rutile dans la modification est préféré.

10. Procédé selon la revendication 1, **caractérisé en ce que** jusqu'à 30 % en masse de CaZr₄(PO₄)₆ ou de CaTi₄(PO₄)₆ ou des mélanges de ceux-ci ou des solutions solides composées de ceux-ci sont ajoutés pour obtenir un ciment radio-opaque.

11. Procédé selon l'une des revendications 1 à 10, **caractérisé en ce que** la porosité du ciment durci est ajustée grâce à la part de matériau biocéramique résorbable.

12. Procédé selon l'une des revendications 1 à 11, **caractérisé en ce que** la viscosité du ciment moulable et injectable est ajustée grâce à la part d'éléments constitutifs de mélange et/ou au poids moléculaire du PMMA.

13. Kit de ciment à os à base de polyméthacrylate de méthyle, **caractérisé par** les composants suivants, présents en étant séparés les uns des autres
a) 15 à 50 % en masse, par rapport au poids total du ciment à os, d'un polyméthacrylate de méthyle (PMMA) exempt de monomère avec une masse moléculaire moyenne de 3000 à 20000 Dalton et un indice d'acidité de 10 à 350 mg KOH par g de polymère;
b) 10 à 40 % en masse, par rapport au poids total du ciment à os, d'un solvant ou d'un mélange de solvant organique, biologiquement compatible pour le PMMA ;
c) 0,05 à 80 % en masse, par rapport au poids total du ciment à os, d'un matériau bioactif, vitro-cristallin, avec une grosseur de grain dans la plage de >20 à 200 µm, constitué de 15 à 45 % en masse de CaO, 40 à 45 % en masse de P₂O₅, 10 à 40 % en masse de ZrO₂ et 0,7 à 3,5 % en masse de fluorure, par rapport au poids total du matériau vitro-cristallin, et comprenant comme phases cristallines principales de l'apatite et du phosphate de zirconium et calcium et comme composant minoritaire une phase vitreuse, dans lesquelles les phases cristallines principales du matériau vitro-cristallin sont conjointement au moins de 35 % en masse et les composants minoritaires de 5 à 15 % en masse.

14. Kit de ciment à os selon la revendication 13, **caractérisé en ce qu'**il contient en outre un composant, sélectionné parmi le groupe constitué du TiO₂, des produits contrastants radiographiques comme e CaZr₄(PO₄)₆ ou le CaTi₄(PO₄)₆, un matériau biocéramique résorbable comprenant des phases cristallines de Ca₂Kna(PO₄)₂ et une structure poreuse ouverte interne, une vitrocéramique stable à long terme à base d'apatite/de wollastonite ou des mélanges de ceux-ci.

15. Kit de ciment à os selon la revendication 13, **caractérisé en ce que** l'ester acétylacétique ou un mélange d'ester acétylacétique à de l'éthanol est le solvant biologiquement compatible, l'éthanol pouvant contenir jusqu'à 4 % en volume d'eau, l'ester acétylacétique étant préféré.
